(19) Europäisches Patentamt
European Patent Office
Office européen des brevets

(11) Veröffentlichungsnummer: 0 341 514 A2

# (12) EUROPÄISCHE PATENTANMELDUNG

(21) Anmeldenummer: 89107740.6

(22) Anmeldetag: 28.04.89

(51) Int. Cl.4: C07C 67/343 , C07C 69/76 , C07C 69/157 , C07C 67/293 , C07C 41/30 , C07C 43/205 , C07C 120/00 , C07C 121/64

(30) Priorität: 11.05.88 DE 3816120

(43) Veröffentlichungstag der Anmeldung: 15.11.89 Patentblatt 89/46

(84) Benannte Vertragsstaaten: CH DE FR GB LI

(71) Anmelder: BAYER AG
D-5090 Leverkusen 1 Bayerwerk(DE)

(72) Erfinder: Himmler, Thomas, Dr.
Bonhoeffer Strasse 20
D-5000 Köln 80(DE)
Erfinder: Braden, Rudolf, Dr.
Nothauser Feld 1
D-5068 Odenthal-Scheuren(DE)

## (54) Verfahren zur Herstellung von unsymmetrischen Biarylverbindungen.

(57) Unsymmetrische Biarylverbindungen werden hergestellt, indem man zwei verschiedene Halogenaromaten durch Umsetzung mit einem Metall kuppelt und die Umsetzung in Gegenwart katalytischer Mengen einer Nickelverbindung, eines Promotors und eines phosphorhaltigen Liganden durchführt.

EP 0 341 514 A2

## Verfahren zur Herstellung von unsymmetrischen Biarylverbindungen

Die vorliegende Erfindung betrifft ein Verfahren zur Herstellung von unsymmetrischen Biarylverbindungen aus einem Chloraromaten und einem Halogenaromaten.

Unsymmetrische Biarylverbindungen sind von technischem Interesse als Vorprodukte für Aufheller, Pharmazeutika, Pflanzenschutzwirkstoffe und flüssig-kristalline Materialien.

T. T. Tson et al., J. A. C. S, 101, 7547-7560 (1979) berichten vom Auftreten unsymmetrischer Biarylverbindungen bei der Kupplung von zwei verschiedenen Arylbromiden oder Aryliodiden mit metallischem Zink und katalytischen Mengen einer Nickelverbindung. Eine Übertragbarkeit dieser Reaktion auf die weniger reaktiven Chloraromaten und positive Einflüsse durch Promotoren können aus dieser Veröffentlichung nicht hergeleitet werden.

Die EP-A-0 012 201 beschreibt die Herstellung symmetrischer Biarylverbindungen durch Kupplung von zwei identischen Chloraromaten mit metallischem Zink in Gegenwart von Nickel, Promotoren und phosphorhaltigen Liganden. In einer parallelen Veröffentlichung der gleichen Autoren (siehe J. Org. Chem. 51, 2627-2637 (1986)) ist ausdrücklich angegeben, daß dieses Verfahren zur Herstellung unsymmetrischer Biarylverbindungen nicht geeignet ist.

Es wurde nun ein Verfahren zur Herstellung von unsymmetrischen Biarylverbindungen gefunden, das dadurch gekennzeichnet ist, daß man einen gegebenenfalls substituierten Chloraromaten mit einem anders substituierten Jod-, Brom- oder Chloraromaten durch Umsetzung mit einem Metall kuppelt und die Umsetzung in Gegenwart katalytischer Mengen einer Nickelverbindung, eines Promotors und eines phosphorhaltigen Liganden durchführt. Vorzugsweise ist der anders substituierte Jod-, Brom- oder Chloraromat ebenfalls ein Chloraromat.

In das erfindungsgemäß Verfahren können beispielsweise Halogenaromaten der Formel (I) eingesetzt werden

Ar - Hal (I),

in der

Hal für Jod, Brom oder Chlor und

Ar für einen gegebenenfalls substituierten, carbocyclischen oder Heteroatome enthaltenden aromatischen Ring mit 5 oder 6 ringbildenden Atomen steht. Als Heteroatome kommen beispielsweise Stickstoff- und/oder Phosphoratome in Frage. Bevorzugte Heteroatome enthaltende aroma tische Ringe mit 5 oder 6 ringbildenden Atomen enthalten 1 bis 2 Stickstoffatome.

Bevorzugte Verbindungen der Formel (I) sind solche der Formeln (II), (III) oder (IV)

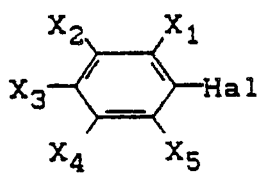

(II)

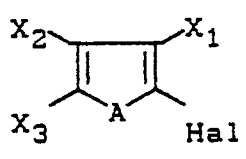

(III)

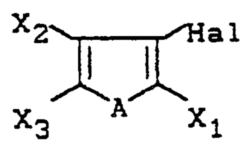

(IV),

in denen

Hal für Jod, Brom oder Chlor stehen und

$X_1$ bis $X_5$ gleich oder verschieden sein können und unabhängig voneinander für Wasserstoff, $C_1$ - bis $C_6$-Alkyl, gegebenenfalls substituiertes $C_6$- bis $C_{14}$-Aryl, Fluor, $C_1$- bis $C_6$-Alkoxy, $C_1$- bis $C_6$-Alkylthio, $C_1$- bis $C_2$-Halogenalkyl mit 1 bis 5 Halogenatomen, $C_1$- bis $C_2$-Halogenalkoxy mit 1 bis 5 Halogenatomen, gegebenenfalls substituiertes $C_6$- bis $C_{14}$-Phenoxy, $C_1$- bis $C_{18}$-Alkylamino, $C_2$- bis $C_{36}$-Dialkylamino, $C_6$-

bis $C_{14}$-Arylamino, $C_{12}$- bis $C_{18}$-Diarylamino, $C_1$- bis $C_{18}$-Carboxyalkyl, $C_6$-bis $C_{10}$-Carboxyaryl, Formyl, Cyano, $C_1$- bis $C_{18}$-Alkyl sulfinyl, $C_1$- bis $C_{18}$-Alkylsulfonyl, $C_6$- bis $C_{14}$-Arylsulfinyl, $C_6$- bis $C_{14}$-Arylsulfonyl, OCO-$C_1$- bis $C_{18}$-Alkyl oder OCO-$C_6$- bis $C_{14}$-Aryl stehen,
wobei OCO-$C_1$- bis $C_{18}$-Alkyl- und OCO-$C_6$- bis $C_{14}$-Arylreste nicht ortho-ständig zum Halogenatom vorliegen können und wobei zwei beliebige benachbarte Reste aus der Gruppe $X_1$ bis $X_5$ gemeinsam auch für eine der Gruppierungen
$-CH_2-CH_2-CH_2-$, $-CH_2-CH_2-CH_2-CH_2-$, $-CH=CH-CH_2-$, $-CH=CH-CH=CH-$, $-O-CH_2-O-$ oder $-O-CH_2-CH_2-O-$ stehen können, in denen jeweils eines, mehrere oder sämtliche Wasserstoffatome gegebenenfalls durch Fluor, eines oder mehrere oder sämtliche Wasserstoffatome der Gruppierungen $-CH_2-CH_2-CH_2-$ und $-CH_2-CH_2-CH_2-CH_2-$ gegebenenfalls auch durch Chlor substituiert sein können und
A für Sauerstoff, Schwefel oder NR, mit R = $C_1$- bis $C_{10}$-Alkyl, Benzyl, gegebenenfalls substituiertes Phenyl mit 6 bis 20 C-Atomen, Formyl oder Acetyl, steht.

Weitere bevorzugte Halogenaromaten sind solche der Formel (II), bei denen wenigstens $X_1$ oder $X_5$ für Wasserstoff steht und solche der Formel (IV), bei denen wenigstens $X_1$ oder $X_2$ für Wasserstoff steht.

$X_1$ bis $X_5$ sind vorzugsweise gleich oder verschieden und stehen unabhängig voneinander für Wasserstoff, $C_1$- bis $C_4$-Alkyl, gegebenenfalls durch Fluor, Cyano, $C_1$- bis $C_4$-Alkyl, Acetoxy, $C_1$- bis $C_3$-Carboxyalkyl oder $C_1$- bis $C_2$-Halogenalkyl mit 1 bis 5 Halogen-, insbesondere Fluoratomen, substituiertes $C_6$- bis $C_{10}$-Aryl, Fluor, $C_1$-bis $C_4$- Alkoxy, $C_1$- bis $C_4$-Alkylthio, $C_1$- bis $C_2$-Halo genalkyl mit 1 bis 5 Fluor- und/oder Chloratomen, $C_1$-bis $C_2$-Halogenalkoxy mit 1 bis 5 Fluor- und/oder Chloratomen, gegebenenfalls durch Fluor, Cyano, $C_1$- bis $C_4$-Alkyl, Acetoxy, $C_1$- bis $C_3$-Carboxyalkyl oder $C_1$- bis $C_2$-Halogenalkyl mit 1 bis 5 Halogen-, insbesondere Fluoratomen substituiertes $C_6$- bis $C_{10}$-Phenoxy, $C_1$- bis $C_4$-Alkylamino, $C_2$- bis $C_8$-Dialkylamino, $C_6$- bis $C_{10}$-Arylamino, $C_{12}$- bis $C_{16}$-Diarylamino, $C_1$- bis $C_6$-Carboxyalkyl, $C_6$- bis $C_8$-Carboxyaryl, Formyl, Cyano, $C_1$- bis $C_6$-Alkylsulfinyl, $C_1$- bis $C_6$-Alkylsulfonyl, $C_6$- bis $C_{10}$-Arylsulfinyl, $C_6$- bis $C_{10}$-Arylsulfonyl, OCO-$C_1$- bis $C_6$-Alkyl oder OCO-$C_6$- bis $C_{10}$-Aryl, wobei bei den beiden letztgenannten Resten die weiter oben beschriebenen Einschränkungen ebenfalls zutreffen,
und wobei zwei beliebige benachbarte Reste aus der Gruppe $X_1$ bis $X_5$ auch gemeinsam für eine der Gruppierungen
$-CH_2-CH_2-CH_2-CH_2-$, $-CH=CH-CH=CH-$ oder $-O-CH_2-CH_2-O-$
stehen, die vorzugsweise unsubstituiert sind.

Besonders bevorzugt sind $X_1$ bis $X_5$ gleich oder verschieden und stehen für Wasserstoff, gegebenenfalls durch Fluor, Cyano, Acetoxy oder $C_1$- bis $C_3$-Carboxyalkyl substituiertes Phenyl, Fluor, $C_1$- bis $C_4$-Alkoxy, $C_1$- bis $C_2$-Halogenalkyl mit 1 bis 5 Halogen-, insbesondere Fluoratomen, $C_2$- bis $C_8$-Dialkylamino, $C_1$- bis $C_6$-Carboxyalkyl, Formyl, Cyano oder OCO-$C_1$- bis -$C_6$-Alkyl.

Weitere bevorzugte Verbindungen der Formeln (II), (III) und (IV) enthalten als $X_1$ bis $X_5$ nur Wasserstoffatome oder 1 bis 2 von Wasserstoff verschiedene Reste oder Gruppierungen.

In den Formeln (III) und (IV) steht A vorzugsweise für Schwefel oder NR, mit R = $C_1$- bis $C_6$-Alkyl, Benzyl, gegebenenfalls substituiertes Phenyl mit 6 bis 12 C-Atomen, Formyl oder Acetyl.

Die Verbindungen der Formel (II) sind gegenüber den Verbindungen der Formeln (III) und (IV) bevorzugt.

In das erfindungsgemäße Verfahren werden sich Chloraromat und ein anders substituierter Halogenaromat, wobei Halogen für Jod, Brom oder Chlor steht, eingesetzt und daraus eine Biarylverbindung erhalten, welche die beiden unterschiedlichen aromatischen Reste enthält. Beispielsweise kann man das erfindungsgemäße Verfahren anhand der folgenden Reaktionsgleichung erläutern:

$$Ar - Cl \quad + \quad Ar' - Hal \longrightarrow Ar - Ar'$$

(I) (Ia) unsymmetrische Biarylverbindung

Hal = J, Br, Cl

Ar und Ar′ haben die oben für Ar angegebene Bedeutung, innerhalb der Ar und Ar′ jedoch verschieden voneinander sind.

Man kann die beiden verschiedenen Halogenaromaten in äquimolaren Mengen einsetzen. Häufig ist es auch vor teilhaft, einen der beiden Halogenaromaten im Überschuß einzusetzen, weil sich dann im allgemeinen der im Unterschuß eingesetzte Halogenaromat selektiver zu der gewünschten unsymmetrischen Biarylverbindung umsetzt. Die Auswahl des Halogenaromaten, den man im Überschuß einsetzt, kann anhand verschiedener Kriterien erfolgen. Beispielsweise kann man den Halogenaromaten im Überschuß einsetzen, der besser und/oder kostengünstiger zugänglich ist, oder den Halogenaromaten, dessen sich als Nebenprodukt bildende entsprechende symmetrische Biarylverbindung leichter aus dem Reaktionsgemisch entfernen läßt. Den im Überschuß eingesetzten Halogenaromaten kann man beispielsweise in einer Menge von 110 bis 1000 Mol-%, bezogen auf den anderen Chloraromaten, einsetzen. Vorzugsweise beträgt diese Menge 150 bis 500 Mol-%, besonders bevorzugt 200 bis 400 Mol-%.

Die eingesetzten Halogenaromaten sind vorzugsweise wasserfrei.

Als Metalle zur Kupplung der beiden Halogenaromaten kommen insbesondere Zink, Mangan und Magnesium in Frage. Bevorzugt ist Zink, insbesondere in Form von Zinkpulver, das besonders bevorzugt in gereinigter Form verwendet wird. Die Reinigung kann beispielsweise so erfolgen, daß man das Zinkpulver nacheinander mit Eisessig, Wasser und Aceton wäscht und anschließend im Vakuum trocknet. Die Menge dieses Metalls kann in weiten Grenzen variieren. Beispielsweise kann man, bezogen auf die Summe der beiden eingesetzten Halogenaromaten, 0,5 bis 10 Moläquivavalente des Metalls einsetzen. Bevorzugt beträgt diese Menge 0,5 bis 5 Moläquivalente.

Als Nickelverbindungen kommen insbesondere wasserfreie Nickelverbindungen in Frage, in denen das Nickel in der Oxidationsstufe 0 vorliegt oder durch Zink, Mangan und/ oder Magnesium durch Reduktion in die Oxidationsstufe 0 überführt werden kann. Beispielsweise seien genannt; Nickel(II)-halogenide, wie Nickel(II)-chlorid, Nickel(II)-bromid und Nickel(II)-iodid, Nickel(II)-sulfat, Nickel(II)-carbonat, Nickel(II)-salze organischer Säuren mit 1 bis 18 C-Atomen wie Nickel(II)-acetat und Nickel(II)-propionat, Nickel(II)-komplexe wie Nickel(II)-acetylacetonat und Dichloro-bis-(triphenylphosphin)-nickel(II) und Nickel(0)-komplexe wie Bis-(cycloocta-1,5-dien)-nickel(0) und Tetrakis-(triphenylphosphin)-nickel(0). Alle diese Nickelverbindungen sind im Handel erhältlich oder auf bekannte Weise herstellbar und gegebenenfalls auf bekannte Weise von gebundenem oder anhaftendem Wasser zu befreien.

Bevorzugt sind die genannten Nickel(II)-halogenide in wasserfreier Form, insbesondere Nickel(II)-chlorid, das beispielsweise aus $NiCl_2$ x $6H_2O$ durch Umsetzung mit Thionylchlorid erhältlich ist.

Die Nickelverbindung kann beispielsweise in einer Menge von 0,1 bis 25 Mol-%, bezogen auf die Summe der beiden eingesetzten Halogenaromaten, eingesetzt werden. Vorzugsweise beträgt diese Menge 1 bis 20 Mol-%, besonders bevorzugt 5 bis 15 Mol-%, Es können auch Gemische aus zwei oder mehr Nickelverbindungen eingesetzt werden.

Als Promotoren kommen insbesondere Alkali-, Erdalkali-, Zink-, Magnesium- und Manganhalogenide in Frage. Bevor zugt sind jeweils die lodide. Der oder die Promotoren können beispielsweise in Mengen von 0,1 bis 1000 Mol-%, bezogen auf die eingesetzte Nickelverbindung, eingesetzt werden. Bevorzugt beträgt diese Menge 1 bis 700 Mol-%, besonders bevorzugt 10 bis 500 Mol-%.

Als phosphorhaltige Liganden kommen insbesondere Triarylphosphine in Frage, beispielsweise solche mit 6 bis 10 C-Atomen pro Aryleinheit. Bevorzugt sind Triphenylphosphin, die Tritolylphosphine und Trinaphthylphosphin. Besonders bevorzugt ist Triphenylphosphin. Der phosphorhaltige Ligand oder die phosphorhaltigen Liganden können beispielsweise in einer Menge von 200 vis 10 000 Mol-%, bezogen auf die eingesetzte Nickelverbindung, eingesetzt werden. Bevorzugt beträgt diese Menge 400 bis 5 000 Mol-%, besonders bevorzugt 500 bis 1 000 Mol-%.

Gegebenenfalls kann zusätzlich zu phosphorhaltigen Liganden eine oder mehrere polycyclische, aromatische Verbindungen mit wenigstens zwei Stickstoffatomen in verschiedenen aromatischen Ringen und insgesamt 10 bis 18 C-Atomen eingesetzt werden. Bevorzugt sind dabei 2,2′-Dipyridyl und 1,10-Phenanthrolin. Polycyclische, aromatische Verbindungen können beispielsweise in Mengen von 50 bis 500 Mol-%, bezogen auf die eingesetzte Nickelverbindung, eingesetzt werden.

Das erfindungsgemäße Verfahren kann in Anwesenheit oder Abwesenheit von Lösungsmitteln durchgeführt werden. Vorzugsweise arbeitet man in Anwesenheit von Lösungsmit teln, weil dann z.B. die Abführung der Reaktionswärme gut kontrollierbar ist. Geeignet sind aprotische, polare Lösungsmittel wie Dimethylformamid, Dimethylacetamid, N-Methyl-caprolactam, N-Methyl-pyrrolidinon, Dimethylsulfoxid und Tetramethylensulfon. Vorzugsweise gelangen Lösungsmittel in getrockneter Form zum Einsatz, beispielsweise mit einem Molekularsieb behandelte und/oder gegebenenfalls in einer Schutzgasatmosphäre destillierte Lösungsmittel.

Man kann das erfindungsgemäße Verfahren beispielsweise bei Temperaturen von 0 bis 250°C durchführen. Bevorzugt sind Temperaturen von 20 bis 200°C, insbesondere solche von 50 bis 150°C.

Es ist vorteilhaft, die erfindungsgemäße Umsetzung in einer inerten Schutzgasatmosphäre durchzuführen. Geeignete Schutzgase sind z.B. Stickstoff, Helium und Argon.

Im allgemeinen führt man das erfindungsgemäße Verfahren bei Normaldruck durch. Man kann aber auch bei erhöhtem oder vermindertem Druck arbeiten, beispielsweise im Bereich von 0,1 bis 10 bar.

Eine beispielhafte Ausführungsform des erfindungsgemäßen Verfahrens besteht darin, daß man zunächst das Metall, die Nickelverbindung, den Promotor, den phosphorhaltigen Liganden, gegebenenfalls eine polycyclische, aromatische Verbindung und gegebenenfalls ein polares, aprotisches Lösungsmittel miteinander vermischt, dieses Gemisch vor legt und dazu die beiden Halogenaromaten getrennt oder als Mischung, gegebenenfalls zusammen mit (weiterem) Lösungsmittel zugibt.

Geeignete Reaktionszeiten für die erfindungsgemäße Umsetzung sind beispielsweise solche im Bereich von 3 Minuten bis 24 Stunden. Vorzugsweise beträgt die Reaktionszeit zwischen 0,1 und 10 Stunden, besonders bevorzugt zwischen 0,5 und 6 Stunden.

Die Aufarbeitung des Reaktionsgemisches kann auf verschiedene Weise erfolgen, indem man darin vorliegende Metallsalze, Nickelverbindungen, Liganden, Nebenprodukte (z.B. symmetrische Biarylverbindungen), sowie gegebenenfalls vorhandene Metalle und gegebenenfalls vorhandenes Lösungsmittel abtrennt. Je nach Art und Menge dieser Bestandteile sind unterschiedliche Aufarbeitungsmethoden bevorzugt. Beispielsweise kann man zunächst filtrieren, dann gegebenenfalls aus dem Filtrat Lösungsmittel durch Einengen im Vakuum entfernen, dann den Rückstand mit einem mit Wasser nicht mischbaren Lösungsmittel (z.B. einem chlorierten Kohlenwasserstoff) aufnehmen, diese organische Phase mit Wasser oder wäßriger Salzsäure ausschütteln, die organische Phase wieder abtrennen, einengen und den dann erhältlichen Rückstand gegebenenfalls durch Umkristallisation oder chromatographische Methoden weiter reinigen.

Das erfindungsgemäße Verfahren gestattet die Herstellung von unsymmetrischen Biarylverbindungen in guten Ausbeu ten und Selektivitäten. Dies ist im Hinblick auf den eingangs geschilderten Stand der Technik außerordentlich überraschend, denn bisher wurde in den betroffenen Fachkreisen die Ansicht vertreten, daß ausgehend von Chloraromaten, unsymmetrische Biarylverbindungen nicht auf brauchbare Weise hergestellt werden können. Beim Einsatz der reaktiveren Brom- und Jodaromaten war zu erwarten, daß diese bevorzugt mit sich selbst reagieren und Reaktionsprodukte mit Chloraromaten nicht in nennenswertem Umfang erhalten werden können.

## Beispiele

### Beispiel 1

#### Herstellung von 4-(3-Trifluormethylphenyl)-benzoesäuremethylester

In einer Stickstoffatmosphäre wurden 33 g Zinkpulver, 33 g Triphenylphosphin, 5 g Natriumiodid, 2,2 g Nickel(II)-chlorid und 200 ml Dimethylformamid gemischt und vorgelegt. Die Mischung wurde eine halbe Stunde bei 50 bis 60 °C gehalten, danach lag eine tief rot-braun gefärbte Suspension vor. Zur dieser wurde innerhalb von einer halben Stunde eine Lösung, enthaltend 20,2 g (0,11 Mol) 3-Chlor-benzotrifluorid, 38,2 g (0,22 Mol) 4-Chlor-benzoesäuremethylester und 100 ml Dimethylformamid, zugetropft. Nach 6 Stunden bei 80 °C wurde das Reaktionsgemisch in 500 ml Chloroform gegeben und filtriert. Der Filterrückstand wurde in 400 ml Toluol aufgekocht und heiß filtriert. Nach Abkühlen wurden 15 g Biphenyl-4,4′-dicarbonsäuredimethylester (50,5 % der Theorie - bezogen auf eingesetzten 4-Chlor-benzoesäuremethylester) mit einem Schmelzpunkt von 213 bis 214 °C erhalten. Das Filtrat wurde mit verdünnter wäßriger Salzsäure und mit Wasser ausgeschüttelt, die organische Phase über Natriumsulfat getrocknet und eingeengt. Der resultierende Rückstand wurde destilliert. Auf diese Weise wurden 6,4 g 3,3′-Bis-(trifluormethyl)-biphenyl (20 % der Theorie - bezogen auf eingesetztes 3-Chlorbenzotrifluorid) und insgesamt 16 g 4-(3-Trifluormethyl phenyl)-benzoesäuremethylester (52 % der Theorie - bezogen auf eingesetztes 3-Chlor-benzotrifluorid) mit einem Siedepunkt von 125 bis 130 °C bei 0,1 bis 0,3 mbar erhalten.

### Beispiel 2

#### Herstellung von 4-(4-Trifluormethyl-phenyl)-benzoesäuremethylester

Es wurde genauso verfahren wie in Beispiel 1, jedoch wurde anstelle von 3-Chlor-benzotrifluorid die gleiche Menge 4-Chlor-benzotrifluorid eingesetzt. Auf diese Weise wurden 16,5 g 4-(4-Trifluormethyl-phenyl)-benzoesäuremethylester erhalten. Das entspricht 53 % der Theorie - bezogen auf eingesetztes 4-Chlor-benzotrifluorid.

Beispiel 3

Herstellung von 4-Acetoxy-4′-methyl-biphenyl

In einer Stickstoffatmosphäre wurden 20 g Zinkstaub, 20 g Triphenylphosphin, 3 g Natriumiodid und 1,3 g Nickel(II)-chlorid in 150 ml Dimethylformamid vorgelegt. Die Mischung wurde eine halbe Stunde bei 60 °C gehalten. Danach wurde eine Lösung, enthaltend 12,3 g (0,1 Mol) 4-Chlortoluol, 17,1 g (0,1 Mol) 4-Chloracetoxybenzol und 50 ml Dimethylformamid, zugetropft. Nach 6 Stunden be 80 °C wurde das Reaktionsgemisch filtriert, das Filtrat eingeengt, in 500 ml Chloroform gelöst und mit Wasser ausgeschüttelt. Nach dem Trocknen der organischen Phase über Natriumsulfat und Abziehen des Lösungsmittels wurde der ölige Rückstand gaschromatographisch analysiert. Die Ausbeute an 4-Acetoxy-4′-methyl-biphenyl betrug 40 % der Theorie.

Beispiel 4

Herstellung von 4-Methoxy-4′-methyl-biphenyl

Es wurde genauso verfahren wie in Beispiel 3 beschrieben, jedoch wurde statt 4-Chloracetoxybenzol 14,3 g (0,1 Mol) 4-Chloranisol eingesetzt. 4-Methoxy-4′-methylbiphenyl wurde nach gaschromatographischer Analyse in einer Ausbeute von 49 % der Theorie erhalten.

Beispiel 5

Herstellung von 4-Acetoxy-4′-cyano-bisphenyl

Es wurde genauso verfahren wie in Beispiel 3 beschrieben, jedoch wurde anstelle von 4-Chlortoluol eine entsprechende Menge 4-Chlor-benzonitril eingesetzt. 4-Acetoxy-4′-cyano-biphenyl wurde gemäß gaschromatographischer Analyse in einer Ausbeute von 42 % der Theorie erhalten.

**Ansprüche**

1. Verfahren zur Herstellung von unsymmetrischen Biarylverbindungen, dadurch gekennzeichnet, daß man einen gegebenenfalls substituierten Chloraromaten mit einem anders substituierten Jod-, Brom- oder Chloraromaten durch Umsetzung mit einem Metall kuppelt und die Umsetzung in Gegenwart katalytischer Mengen einer Nickelverbindung, eines Promotors und eines phosphorhaltigen Liganden durchführt.

2. Verfahren nach Anspruch 1, dadurch gekennzeichnet, daß man Halogenaromaten einsetzt, die der Formel (I) entsprechen

Ar - Hal (I),

in der

Hal für Jod, Brom oder Chlor und

Ar für einen gegebenenfalls substituierten, carbocyclischen oder Heteroatome enthaltenden aromatischen Ring mit 5 oder 6 ringbildenden Atomen steht.

3. Verfahren nach Ansprüchen 1 und 2, dadurch gekennzeichnet, daß man Halogenaromaten der Formeln (II), (III) und/oder (IV) einsetzt

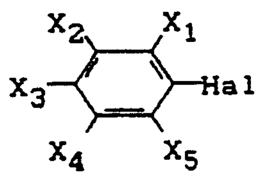

(II)

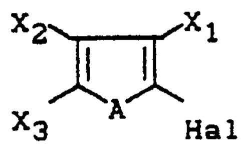

(III)

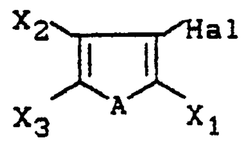

(IV),

in denen
Hal für Jod, Brom oder Chlor stehen und
$X_1$ bis $X_5$ gleich oder verschieden sein können und unabhängig voneinander für Wasserstoff, $C_1$- bis $C_6$-Alkyl, gegebenenfalls substituiertes $C_6$- bis $C_{14}$-Aryl, Fluor, $C_1$- bis $C_6$-Alkoxy, $C_1$- bis $C_6$-Alkylthio, $C_1$- bis $C_2$-Halogenalkyl mit 1 bis 5 Halogenatomen, $C_1$- bis $C_2$-Halogenalkoxy mit 1 bis 5 Halogenatomen, gegebenenfalls substituiertes $C_6$-bis $C_{14}$-Phenoxy, $C_1$- bis $C_{18}$-Alkylamino, $C_2$- bis $C_{36}$-Dialkylamino, $C_6$-bis $C_{14}$-Arylamino, $C_{12}$- bis $C_{18}$-Diarylamino, $C_1$- bis $C_{18}$-Carboxyalkyl, $C_6$-bis $C_{10}$-Carboxyaryl, Formyl, Cyano, $C_1$- bis $C_{18}$-Alkylsulfinyl, $C_1$- bis $C_{18}$-Alkylsulfonyl, $C_6$- bis $C_{14}$-Arylsulfinyl, $C_6$- bis $C_{14}$-Arylsulfonyl, OCO-$C_1$- bis $C_{18}$-Alkyl oder OCO-$C_6$- bis $C_{14}$-Aryl stehen,
wobei OCO-$C_1$- bis $C_{18}$-Alkyl- und OCO-$C_6$- bis $C_{14}$-Arylreste nicht ortho-ständig zum Halogenatom vorliegen können
und wobei zwei beliebige benachbarte Reste aus der Gruppe $X_1$ bis $X_5$ gemeinsam auch für eine der Gruppierungen $-CH_2-CH_2-CH_2-$, $CH_2-CH_2-CH_2-CH_2-$, $-CH=CH-CH_2-$, $-CH=CH-CH=CH-$, $-O-CH_2-O-$ oder $-O-CH_2-CH_2-O-$ stehen können, in denen jeweils eines, mehrere oder sämtliche Wasserstoffatome gegebenenfalls durch Fluor, eines oder mehrerer oder sämtlicher Wasserstoffatome der Gruppierungen $-CH_2-CH_2-CH_2-$und $-CH_2-CH_2-CH_2-CH_2-$, gegebenenfalls auch durch Chlor substituiert sein können und
A für Sauerstoff, Schwefel oder NR, mit R = $C_1$- bis $C_{10}$-Alkyl, Benzyl, gegebenenfalls substituiertes Phenyl mit 6 bis 20 C-Atomen, Formyl oder Acetyl, steht.

4. Verfahren nach Ansprüchen 1 bis 3, dadurch gekennzeichnet, daß man einen der Halogenaromaten in einer Menge von 110 bis 1000 Mol-%, bezogen auf den anderen Chloraromaten, einsetzt.

5. Verfahren nach Ansprüchen 1 bis 4, dadurch gekennzeichnet, daß man als Metall zur Kupplung der zwei verschiedenen Halogenaromaten Zink, Mangan und/oder Magnesium in einer Menge von 0,5 bis 10 Moläquivalenten, bezogen auf die Summe der beiden Halogenaromaten, einsetzt.

6. Verfahren nach Ansprüchen 1 bis 5, dadurch gekennzeichnet, daß man eine wasserfreie Nickelverbindung einsetzt, in der das Nickel in der Oxidationsstufe 0 vorliegt oder durch Zink, Mangan und/oder Magnesium durch Reduktion in die Oxidationsstufe 0 überführt werden kann in einer Menge von 0,1 bis 25 Mol-%, bezogen auf die Summe der beiden eingesetzten Halogenaromaten.

7. Verfahren nach Ansprüchen 1 bis 6, dadurch gekennzeichnet, daß man als Promotoren Alkali-, Erdalkali-, Zink-, Magnesium- und/oder Manganhalogenide in einer Menge von 0,1 bis 1000 Mol-%, bezogen auf die eingesetzte Nickelverbindung, einsetzt.

8. Verfahren nach Ansprüchen 1 bis 7, dadurch gekennzeichnet, daß man als phosphorhaltige Liganden Triarylphosphine in einer Menge von 200 bis 10 000 Mol-%, bezogen auf die eingesetzte Nickelverbindung, einsetzt.

9. Verfahren nach Ansprüchen 1 bis 8, dadurch gekennzeichnet, daß man zusätzlich zu phosphorhaltigen Liganden eine oder mehrere polycyclische, aromatische Verbindungen mit wenigstens zwei Stickstoffatomen in verschiedenen aromatischen Ringen und insgesamt 10 bis 18 C-Atomen in einer Menge von 50 bis 500 Mol-%, bezogen auf die eingesetzte Nickelverbindung, einsetzt.

10. Verfahren nach Ansprüchen 1 bis 9, dadurch gekennzeichnet, daß man es in Gegenwart eines aprotischen, polaren Lösungsmittels und bei Temperaturen im Bereich 0 bis 250 °C durchführt.